(19) Europäisches Patentamt
European Patent Office
Office européen des brevets

(11) Veröffentlichungsnummer: **0 093 853 A1**

(12) # EUROPÄISCHE PATENTANMELDUNG

(21) Anmeldenummer: **83102406.2**

(22) Anmeldetag: **11.03.83**

(51) Int. Cl.³: **C 07 C 49/395,** C 07 C 43/305, C 07 D 317/72, C 07 C 41/56, C 07 C 45/54, A 61 K 7/46, C 11 B 9/00

(30) Priorität: **30.04.82 DE 3216085**

(43) Veröffentlichungstag der Anmeldung: **16.11.83**
**Patentblatt 83/46**

(84) Benannte Vertragsstaaten: **AT CH DE FR GB LI NL**

(71) Anmelder: **CHEMISCHE WERKE HÜLS AG, - RSP Patente / PB 15 - Postfach 13 20, D-4370 Marl 1 (DE)**

(72) Erfinder: **Burzin, Klaus, Dr., Wellerfeldweg 164, D-4370 Marl (DE)**

(54) **Substituierte 3-Isopropyl-cyclopentanonderivate, deren Herstellung und Verwendung als Riechstoffe sowie diese enthaltende Riechstoffkompositionen.**

(57) Die Erfindung betrifft die Verwendung von 3-Isopropyl-cyclopentanonderivaten der allgemeinen Formel

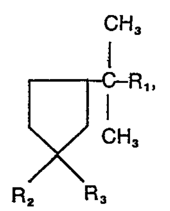

wobei $R_1$ Wasserstoff oder ein gegebenenfalls verzweigter Alkylrest mit 1 bis 5 C-Atomen ist und $R_2$ und $R_3$ zusammen entweder den doppelt gebundenen Sauerstoff einer Ketogruppe oder ein Ethylenketal bezeichnen, als Riechstoffe.

EP 0 093 853 A1

Substituierte 3-Isopropyl-cyclopentanonderivate, deren Herstellung und Verwendung als Riechstoffe sowie diese enthaltende Riechstoffkompositionen

Es wurde gefunden, daß 3-Isopropyl-cyclopentanonderivate der allgemeinen Formel

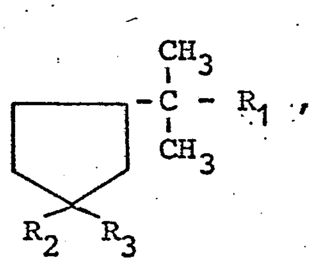

wobei $R_1$ Wasserstoff oder ein gegebenenfalls verzweigter Alkylrest mit 1 bis 5 C-Atomen ist und $R_2$ und $R_3$ zusammen entweder den doppelt gebundenen Sauerstoff einer Ketogruppe oder ein Ethylenketal bezeichnen, wertvolle Riechstoffe mit intensiver holziger Geruchsnote darstellen, die mit Ausnahme des 3-Isopropyl-, 3-tert.-Butyl- und des 3-tert.-Amylcyclopentanon neu sind.

Gegenstand der Erfindung ist ferner ein Verfahren zur Herstellung der bisher unbekannten 2-Isopropyl-cyclopentanonderivate.

Alkylsubstituierte Cyclopentanone, ihre Ethylenketale und Verfahren zu ihrer Herstellung sind an sich bekannt.

Beispielsweise haben stereochemische Untersuchungen zur Synthese von 2-Hydroxymethyl-2-methylcyclopentanon und seines Ethylenketals /J. Liebigs Annalen der Chemie 580, 132 (1953)/ sowie zur Herstellung von 3-tert.-Butyl- und 3-tert.-Amylcyclopentanon /JACS 61, 2728 (1939)/ geführt. Im Rahmen einer Konstitutionsaufklärung wurde 3-Isopropylcyclopentanon synthetisiert (J. Chem. Soc. 1946, 145). Hinweise, daß die Cyclopentanone Riechstoffeigenschaften besitzen, werden in diesen Publikationen nicht gegeben.

2,3-disubstituierte Cyclopentanone finden generell als Arzneimittel, landwirtschaftliche Chemikalien sowie als Parfüme auf Jasminbasis /vgl. J. Org. Chem. 39, 2637 (1974)/ Verwendung. Die DE-OS 24 55 444 beschreibt ein Verfahren zur Herstellung dieser Verbindungen durch Addition von organischen Kupferlithiumverbindungen an α,ß-ungesättigte Cyclopentanone. Verschiedene 3-Methyl-2-pentylcyclopentanone und ihre Ethylenketale mit blumiger Geruchsnote werden in der DE-OS 23 01 854 beschrieben. Die 2-Alkyl-1,4-dioxaspiro(4,n)-alkane der DE-A1-29 45 049 der allgemeinen Formel

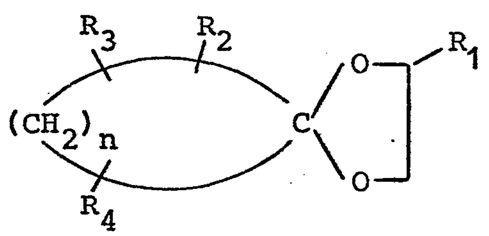

$R_1$ = n-Alk ($C_4$-$C_{12}$)

$R_2$, $R_3$, $R_4$ = H, Alk ($C_1$-$C_4$)

n = 4, 5, 6

stellen Riechstoffe mit blumiger Geruchsnote dar. 2-Isopropyl-4-methylcyclopentanon und 2,4,4-Trimethylcyclopentanon weisen Pfefferminznoten auf /vgl. Seifen-Öle-Fette-Wachse, 108, 12 (1982)/. Auch das in der EP-A3-0 016 650 beschriebene 2-Cyclopentylcyclopentanon zeichnet sich durch einen frischen kühlen Geruch aus.

Der Literatur läßt sich indessen kein Hinweis auf die besondere Eignung von 3-Isopropyl-cyclopentanonen und ihrer Ethylenketale als Riechstoffe mit kräftiger holziger bzw. holzig-krautiger oder holzig-würziger Geruchsnote entnehmen, die eine sehr gute Kombinationsfähigkeit zu neuen, interessanten Kompositionen besitzen.

Andererseits besteht schon seit einiger Zeit ein Bedürfnis nach Riechstoffen mit intensiver holziger Geruchsnote. Die in der Literatur bekannten Cyclododecylderivate der Formel

0093853
O.Z.3810

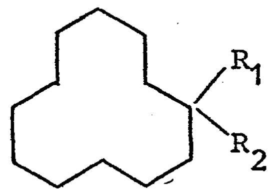

$R_1$= H, $R_2$= CH-O-Alk (DE-PS 29 28 347)
$R_1$= H, $R_2$= O-Alk (CH-PS 443 536)
$R_1$= $CH_3$, $R_2$= O-Alk (DE-B2-21 52 016)

deren holzige Geruchsnote bekannt ist, weisen bei Raumtemperatur eine für spezielle Einsatzzwecke - wie z. B. die Parfümierung von kosmetischen Artikeln - in der Regel nicht ausreichende Geruchsintensität auf.

Die erfindungsgemäßen Riechstoffe zeichnen sich dagegen durch kräftige, vorwiegend holzige Geruchsnoten aus. Sie können mit anderen Riechstoffen in den verschiedensten Mengenverhältnissen gemischt werden. Im allgemeinen beträgt der Anteil der erfindungsgemäßen Riechstoffe 1 bis 50 Gewichtsprozent, bezogen auf die gesamte Riechstoffkomposition.

Derartige Kompositionen werden zur Parfümierung von kosmetischen Präparaten, wie Cremes, Lotionen, Duftwässern, Aerosolen, Seifen, als auch bei der Herstellung von Extrait-Parfüms verwendet.

In Konzentrationen von 0,05 bis etwa 5 Gewichtsprozent, bezogen auf das gesamte Produkt, können die erfindungsgemäßen Riechstoffe auch zur Geruchsverbesserung technischer Produkte, wie Wasch-, Reinigungs- und Weichspülmittel eingesetzt werden.

Die Herstellung der erfindungsgemäßen Riechstoffe erfolgt nach an sich bekannten Syntheseverfahren der organischen Chemie. Als Ausgangsmaterial für die Synthese dienen 3-Isopropyladipinsäuren der Formel

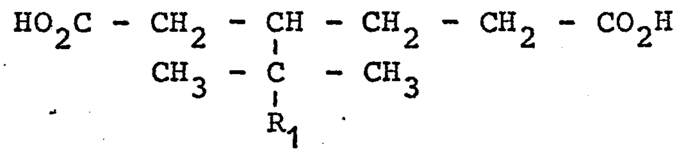

$R_1$= H, Alk ($C_1$-$C_5$)

Diese können durch Alkylierung von Phenol, Reduktion zu 4-alkylsubstituierten Cyclohexanolen und anschließende Oxidation hergestellt werden /Zh. Prikl. Khim. 39 (3), 668 - 671 (1966) bzw. JACS 59, 717 (1937)/. Die Überführung der substituierten 3-Isopropyladipinsäuren in substituierte 3-Isopropyl-cyclopentanone erfolgt durch Erhitzen der Adipinsäuren in Gegenwart von Calcium- oder Bariumsalzen, wie es z. B. in Org. Synth. Coll. Vol. I 192 (1948) beschrieben ist. Die Ketalisierung wurde nach einer Literaturvorschrift durchgeführt (vgl. Organikum Seite 431, VEB Deutscher Verlag der Wissenschaften, Berlin 1970).

Beispiele

1. Herstellung der substituierten 3-Isopropyl-cyclopentanone

100 g der substituierten 3-Isopropyladipinsäure wurden in einer Destillationsapparatur zusammen mit 10 g Bariumhydroxid auf 280 bis 300 °C erhitzt. Innerhalb von 2 Stunden bildete sich das jeweilige substituierte 3-Isopropyl-cyclopentanon. Nach der Abtrennung von Wasser wurde mit $K_2CO_3$-Lösung gewaschen, über $Na_2SO_4$ getrocknet und danach fraktioniert.

Nach dieser Vorschrift wurden folgende substituierte 3-Isopropyl-cyclopentanone hergestellt:

1.1 3-Isopropyl-cyclopentanon
Siedepunkt: 81 °C (17 mbar)
Ausbeute : 72 %
Geruch : holzig-stechend

1.2 3-tert.-Butylcyclopentanon
Siedepunkt: 84 °C (1,5 mbar)
Ausbeute : 74 %
Geruch : bittere Nußnote

1.3 3-/(2,4-Dimethyl)-2-pentyl/-cyclopentanon
Siedepunkt: 112 °C (14 mbar)
Ausbeute : 68 %
Geruch : holzig-würzig

1.4 3-/(2,4,4-Trimethyl)-pentyl/-cyclopentanon
Siedepunkt: 138 °C (15 mbar)
Ausbeute : 76 %
Geruch : holzig-süß

2. Herstellung der substituierten 3-Isopropyl-cyclopentanonethylenketale

1 Mol Keton wurde mit 1,2 Mol Ethylenglykol und 0,1 g p-Toluolsulfonsäure in 150 ml Toluol am Wasserabscheider unter Rückfluß erhitzt, bis sich kein Reaktionswasser mehr gebildet hat. Danach wurde abgekühlt, sorgfältig mit verdünnter NaOH und Wasser gewaschen, mit Kaliumcarbonat getrocknet und destilliert.

2.1 3-Isopropyl-cyclopentanonethylenketal
Siedepunkt: 49 °C (0,5 mbar)
Ausbeute : 66 %
Geruch : holzig, Eucalyptus-Note

2.2 3-tert.-Butylcyclopentanonethylenketal
Siedepunkt: 51 °C (0,3 mbar)
Ausbeute : 86 %
Geruch : holzig-blumig

2.3 3-/(2,4-Dimethyl)-2-pentyl/-cyclopentanonethylenketal

Siedepunkt: 66 °C (0,2 mbar)
Ausbeute : 72 %
Geruch : holzig-süß

2.4 3-/(2,4,4-Trimethyl)-2-pentyl/-cyclopentanonethylenketal

Siedepunkt: 92 °C (0,2 mbar)
Ausbeute : 81 %
Geruch : holzig-blumig, süß

Anschließend folgen zwei Beispiele von Riechstoffkompositionen.

Beispiel A (Holzbase)

| | Gewichtsteile |
|---|---|
| 3-/(2,4,4-Trimethyl)-2-pentyl/-cyclopentanon | 200 |
| Vetiverylacetat | 100 |
| Isoraldein 70 | 50 |
| Guajylacetat | 50 |
| Zedernketon | 150 |
| Cumarin | 50 |
| Oryclon | 150 |
| Phenylethylalkohol | 100 |
| 1,4-Dioxa-cyclohexadecan-5,16-dion | 150 |
| | 1 000 |

Beispiel B (Phantasiekomposition, blumenartig)

| | Gewichtsteile |
|---|---|
| 3-tert.-Butylcyclopentanonethylenketal | 150 |
| Phenylethylalkohol | 50 |
| Cumarin | 100 |
| Ylang (synth.) | 100 |
| Citronellol | 100 |
| Bergamotte (synth.) | 50 |

| | |
|---|---|
| Benzylacetat | 50 |
| Linalylacetat | 150 |
| Linalool | 200 |
| 1,4-Dioxacyclohexadecan-5,16-dion | 50 |
| | 1 000 |

0093853
O.Z. 3810

Patentansprüche:

1. Verwendung von 3-Isopropyl-cyclopentanonderivaten der allgemeinen Formel

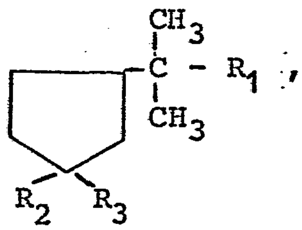

wobei $R_1$ Wasserstoff oder ein gegebenenfalls verzweigter Alkylrest mit 1 bis 5 C-Atomen ist und $R_2$ und $R_3$ zusammen entweder den doppelt gebundenen Sauerstoff einer Ketogruppe oder ein Ethylenketal bezeichnen, als Riechstoff.

2. 3-Isopropyl-cyclopentanonderivate gemäß Anspruch 1, wobei $R_1$ ein gegebenenfalls verzweigter Alkylrest mit 3 bis 5 C-Atomen ist und $R_2$ und $R_3$ zusammen den doppelt gebundenen Sauerstoff einer Ketogruppe bezeichnen.

3. 3-Isopropyl-cyclopentanonderivate gemäß Anspruch 1, wobei $R_1$ ein gegebenenfalls verzweigter Alkylrest mit 1 bis 5 C-Atomen ist und $R_2$ und $R_3$ zusammen ein Ethylenketal bezeichnen.

4. Verfahren zur Herstellung der Verbindungen nach den Ansprüchen 2 und 3,
dadurch gekennzeichnet,
daß man in an sich bekannter Weise die entsprechenden 3-Isopropyladipinsäuren in Gegenwart von Calcium- oder Bariumsalzen erhitzt und gegebenenfalls die entstandenen 3-Isopropyl-cyclopentanone am Wasserabscheider mit Ethylenglykol kocht und damit in die entsprechenden Ethylenketale überführt.

Europäisches Patentamt

# EUROPÄISCHER RECHERCHENBERICHT

0093853

Nummer der Anmeldung

EP 83 10 2406

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | Chemical Abstracts Band 59, Nr. 4, 19. August 1963, Columbus, Ohio, USA T. ONOGAKI "2-Isopropylcyclopentanone from Japanese mint oil in Hokkaido", Spalte 3780e-f & Nippon Kagaku Zasshi, Band 83, 1962, Seiten 206-208 | 1 | C 07 C 49/395<br>C 07 C 43/305<br>C 07 D 317/72<br>C 07 C 41/56<br>C 07 C 45/54<br>A 61 K 7/46<br>C 11 B 9/00 |
| A | Chemical Abstracts Band 65, Nr. 4, 15. August 1966, Columbus, Ohio, USA N.K. TAIKOVA et al. "Synthesis of some 3-substituted adiponitriles", Spalte 5362a-c & Zh. Prik. Khim, Band 39, Nr. 3, 1966, Seiten 668-671 (Kat. D) | 4 | |
| A | DE-B-2 721 002 (FIRMENICH) * Ansprüche 1, 2 * | 1 | RECHERCHIERTE SACHGEBIETE (Int. Cl. 3) |
| A,D | DE-A-2 945 049 (HENKEL) * Ansprüche 1, 5 * | 1 | A 61 K 7/46<br>C 07 C 41/56<br>C 07 C 43/305<br>C 07 C 45/54<br>C 07 C 49/395<br>C 07 D 317/72<br>C 11 B 9/00 |

Der vorliegende Recherchenbericht wurde für alle Patentansprüche erstellt.

| Recherchenort | Abschlußdatum der Recherche | Prüfer |
|---|---|---|
| BERLIN | 02-08-1983 | KNAACK M |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82